# EUROPÄISCHE PATENTANMELDUNG

(11) **EP 0 578 016 A2**
(43) Veröffentlichungstag der Anmeldung: **12.01.1994**
(21) Anmeldenummer: 93109597.0
(22) Anmeldetag: 16.06.1993
(51) Int. Cl.: C12P 41/00, C12P 17/06, C07D 309/30, C07D 305/12

(54) **Enzymatisches Verfahren zur Trennung racemischer Gemische von delta-Valerolactonen**

(30) Priorität: 06.07.1992 AT 1374/92
(71) Anmelder: Chemie Linz GmbH, A-4021 Linz (AT)
(72) Erfinder: Pöchlauer, Peter, Dr., A-4020 Linz (AT); Wagner, Marion, Dipl.-Ing., A-4223 Katsdorf (AT)
(74) Vertreter: Kunz, Ekkehard, Dr.

(57) **Zusammenfassung**

Verfahren zur Trennung racemischer Gemische von beta-Hydroxy- oder beta-Acyloxy-delta-valerolactonen, die in Position 2 und/oder 5 des Lactonringes durch Wasserstoff, Alkylgruppen mit 4 bis 20 C-Atomen, die gegebenenfalls durch ein Sauerstoffatom unterbrochen sind oder durch Aralkylgruppen substituiert sind, wobei nicht beide Positionen durch Wasserstoff substituiert sind, durch Reaktion des racemischen Gemisches mit Hilfe einer Hydrolase mit oder ohne Veresterungsmittel in einem Verdünnungsmittel und Auftrennen des Reaktionsgemisches, das ein enantiomerenreines beta-Hydroxy-delta-valerolacton und ein enantiomerenreines beta-Acyloxy-delta-valerolacton enthält, durch übliche Methoden in die enantiomerenreinen Verbindungen, die Verwendung des Verfahrens zur Herstellung enantiomerenreiner Oxetanone und enantiomerenreine beta-Acyloxy-delta-valerolactone.

## Beschreibung

In EP-A-0 443 449 ist beschrieben, daß insbesondere bestimmte Enantiomere von Oxetanonen, die durch Alkylketten oder Arylgruppen substituiert sind, gute pharmazeutische Wirkungen entfalten.

Zur Herstellung dieser enantiomerenreinen Oxetanone sind in EP-A-0 443 449 zwei Reaktionsfolgen beschrieben.
Gemäß Reaktionsfolge 1 wird ausgehend von Acetessigsäuremethylester eine racemische Mischung eines (2RS, 3RS, 5SR)-2-Hexyl-3-hydroxy-5-undecyl-delta-valerolactons als Zwischenprodukt hergestellt, das über mehrere Stufen zu rac-(2RS, 3RS, 5SR)-5-Benzyloxy-2-hexyl-3-hydroxyhexadecansäure umgesetzt wird. Auf dieser Stufe erfolgt eine Racematspaltung durch Salzbildung mit Hilfe eines enantiomerenreinen Amins, wodurch das (2S, 3S, 5R)-Enantiomer vom (2R, 3R, 5S)-Enantiomer abgetrennt wird. Anschließend wird die enantiomerenreine (2S, 3S, 5R)-Verbindung zum enantiomerenreinen Oxetanon cyclisiert. Da bei dieser Reaktionsfolge die Racematspaltung sehr spät erfolgt, muß das für die Herstellung der enantiomerenreinen Oxetanone unbrauchbare Enantiomer über viele Stufen mitumgesetzt werden, bevor es endgültig verworfen wird.
Gemäß Reaktionsfolge 2 wird enantiomerenreiner 3-Hydroxytetradecansäureester über mehrere Stufen zum enantiomerenreinen 5-((R)-3-Benzyloxy-1-hydroxytetradecyliden)-2,2-dimethyl-m-dioxan-4,6-dion umgesetzt, das racemisierungsfrei zum (R)-5,6-Dihydro-6-undecyl-2H-pyran-2,4(3H)-dion cyclisiert und über mehrere Stufen weiter zum enantiomerenreinen Oxetanon reagieren gelassen wird. Dabei muß aber enantiomerenreiner 3-Hydroxytetradecansäureester, der nicht einfach herzustellen ist, als Ausgangsprodukt verwendet werden. Da die oben beschriebene Cyclisierung zum Pyran in sehr schlechten Ausbeuten verläuft, gehen bei dieser Reaktionsfolge große Mengen an enantiomerenreinem Material verloren.

Es wurde nun gefunden, daß man zur Herstellung enantiomerenreiner Oxetanone gemäß der EP-A-0 443 449 die Racematspaltung schon in einer frühen Verfahrensstufe der Reaktionsfolge 1 auf sehr einfache und effektive Weise durchführen kann, wobei die oben beschriebene Cyclisierung zum Pyran vermieden wird.

Wird ein wie oben beschriebenes racemisches Gemisch von beta-Hydroxy-delta-valerolactonen bzw. von beta-Acyloxy-delta-valerolactonen unter Verwendung einer Hydrolase selektiv verestert bzw. hydrolysiert, erhält man auf einfache Weise eine Mischung eines enantiomerenreinen (2S, 3S, 5R)-beta-Hydroxy-delta-valerolactons mit einem enantiomerenreinen (2R, 3R, 5S)-beta-Acyloxy-delta-valerolacton bzw. eine Mischung eines enantiomerenreinen (2R, 3R, 5S)-beta-Hydroxy-delta-valerolactons mit einem enantiomerenreinen (2S, 3S, 5R)-beta-Acyloxy-delta-valerolacton, die dann auf einfache Weise getrennt werden kann. Dabei kommt die Reaktion nach Umsetzung des einen Enantiomeren erstaunlicherweise völlig zum Erliegen, sodaß Produkte höchster Reinheit gewonnen werden. Die gewünschten enantiomerenreinen Oxetanone können, etwa nach der in EP-A-0 443 449 beschriebenen Methode, aus den abgetrennten Verbindungen, gegebenenfalls nach Abspalten der Acylgruppe, erhalten werden.

Die enzymatische Reaktion ist dabei in extrem kurzer Zeit abgeschlossen. Dies war völlig unerwartet, denn in EP-A-0 439 779 ist beschrieben, daß die enzymatische Trennung von alpha- oder beta-Hydroxy-delta-valerolactonen, die unsubstituiert oder durch Methylgruppen im Lactonring substituiert sind, mit Hilfe der Veresterung der Hydroxygruppe mit einer Lipase und einem Vinylester, bis zu 150 Stunden beträgt. Demgegenüber ist die erfindungsgemäße Reaktion im allgemeinen bereits innerhalb weniger Stunden, in manchen Fällen sogar schon innerhalb von 1 bis 2 Stunden abgeschlossen, obwohl die beta-Hydroxy-valerolactone gemäß vorliegender Erfindung durch Alkylketten oder Arylgruppen substituiert sind, sodaß die Reaktion aus sterischen Gründen noch weitaus langsamer ablaufen müßte, als jene, die in EP-A-O 439 779 beschrieben ist.

Gegenstand der Erfindung ist daher ein Verfahren zur Trennung von racemischen Gemischen einer Verbindung der allgemeinen Formel 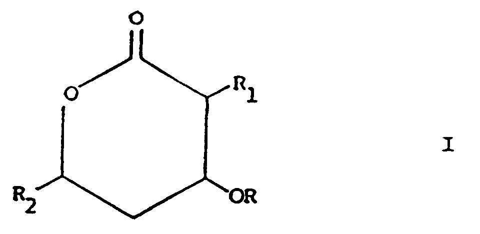
in der R Wasserstoff oder eine Acylgruppe und R₁ und R₂ unabhängig voneinander Wasserstoff, eine geradkettige oder verzweigte Alkylgruppe mit 4 bis 20 C-Atomen, die in einer anderen als der alpha- oder beta-Stellung durch ein Sauerstoffatom unterbrochen sein kann oder eine unsubstituierte oder durch unter den Reaktionsbedingungen inerte Gruppen substituierte Aralkylgruppe bedeuten, mit der Maßgabe, daß R₁ und R₂ nicht gleichzeitig Wasserstoff bedeuten, das dadurch gekennzeichnet ist, daß das racemische Gemisch einer Verbindung der allgemeinen Formel I in einem Verdünnungsmittel vorgelegt und in Gegenwart einer Hydrolase und im Fall, daß R in der allgemeinen Formel I Wasserstoff bedeutet, in Gegenwart eines Veresterungsmittels reagieren gelassen wird, wobei ein Reaktionsgemisch entsteht, das ein enantiomerenreines beta-Hydroxy-delta-valerolacton und ein enantiomerenreines beta-Acyloxy-delta-valerolacton enthält, das auf übliche Art und Weise aufgetrennt wird.

Unter racemisches Gemisch der allgemeinen Formel I sind nicht nur Gemische, die die Enantiomere im Verhältnis von 1:1 enthalten, sondern auch Gemische, die die Enantiomere in beliebiger Zusammensetzung enthalten, in denen also eines der Enantiomeren angereichert vorliegt, zu verstehen.

In der allgemeinen Formel I bedeutet R Wasserstoff oder eine Acylgruppe, bevorzugt Wasserstoff. Eine Acylgruppe ist eine Gruppe der allgemeinen Formel -CO-R₃, in der R₃ eine gegebenenfalls substituierte Alkyl- oder Arylgruppe, bevorzugt eine unsubstituierte Alkylgruppe mit 1 bis 6 C-Atomen, ganz bevorzugt mit 1 bis 4 C-Atomen, bedeutet.
R₁ und R₂ bedeuten unabhängig voneinander Wasserstoff, wobei R₁ und R₂ nicht gleichzeitig Wasserstoff bedeuten, eine Alkylgruppe mit 4 bis 20, bevorzugt mit 4 bis 17 C-Atomen, die geradkettig oder verzweigt, bevorzugt aber geradkettig ist, eine Alkylgruppe mit 4 bis 20, bevorzugt mit 4 bis 17 C-Atomen, die geradkettig oder verzweigt und die in einer anderen Stellung als der alpha- oder beta-Stellung durch ein Sauerstoffatom durchbrochen ist oder eine unsubstituierte, oder durch Alkyl- oder Alkoxygruppen substituierte Aralkylgruppe, wobei die Alkyl- oder Alkoxygruppen bevorzugt 1 bis 6 C-Atome aufweisen. Bevorzugt ist als Aralkylgruppe eine Benzylgruppe. Bevorzugt bedeuten R₁ und R₂ unabhängig voneinander Wasserstoff oder Alkylgruppen, besonders bevorzugt bedeuten R₁ und R₂ Alkylgruppen.
Ein ganz besonders bevorzugtes beta-Hydroxy-delta-valerolacton ist ein solches der allgemeinen Formel I, in der R Wasserstoff oder eine Acylgruppe, R₁ eine Alkylgruppe mit 4 bis 17 C-Atomen und R₂ eine Alkylgruppe mit 6 bis 17 C-Atomen bedeutet.

Die Herstellung von racemischen Gemischen einer Verbindung der allgemeinen Formel I, in der R Wasserstoff bedeutet, kann etwa nach einer der in EP-A-0 443 449 geoffenbarten Verfahrensweise erfolgen. Racemische Gemische einer Verbindung, in der R eine Acylgruppe bedeutet, können mit Hilfe von Veresterungsverfahren, durch die die Gruppe R eingeführt werden kann, aus dem racemischen Gemisch der Verbindungen der allgemeinen Formel I, in der R Wasserstoff bedeutet, hergestellt werden. Bevorzugt erfolgt die Veresterung durch Umsetzung eines racemischen Gemisches einer Verbindung der allgemeinen Formel I, in der R Wasserstoff bedeutet, mit einem Carbonsäureanhydrid oder Carbonsäurechlorid in Gegenwart von Basen wie Pyridin, Triäthylamin, Dimethylaminopyridin.

Zur Durchführung des erfindungsgemäßen Verfahrens wird ein racemisches Gemisch einer Verbindung der allgemeinen Formel I in einem Verdünnungsmittel vorgelegt. Für den Fall, daß R eine Acylgruppe bedeutet, wird kein Veresterungsmittel zugesetzt. Als Verdünnungsmittel wird in diesem Fall Wasser oder eine wäßrige Salz- oder Pufferlösung, bevorzugt ein Phosphatpuffer, der einen pH-Wert aufweist, der für die verwendete Esterase optimal ist, verwendet. Die Pufferlösung kann als solche oder zusammen mit organischen Verdünnungsmitteln eingesetzt werden. Als organische Verdünnungsmittel kommen beispielsweise aliphatische oder aromatische Kohlenwasserstoffe wie Hexan, Toluol, Xylole, Äther, wie Diäthyläther, Diisopropyläther, tert.-Butyl-methyläther, Tetrahydrofuran, Ketone wie Aceton, Butanon, tert.-Butyl-methylketon oder Mischungen solcher Verdünnungsmitteln in Betracht. Durch Zugabe des organischen Verdünnungsmittel zu der Pufferlösung wird eine Anlösung oder Auflösung des racemischen Ausgangsgemisches erreicht. Ist das organische Verdünnungsmittel nicht mit Wasser mischbar, verläuft das erfindungsgemäße Verfahren als 2-Phasenreaktion, sodaß in diesem Fall für eine gute Durchmischung der Phasen gesorgt wird.
Für den Fall daß R Wasserstoff bedeutet, wird der racemischen Ausgangsmischung ein Veresterungsmittel zugesetzt. Als Veresterungsmittel können übliche Veresterungsmittel wie Carbonsäureester, etwa Verbindungen der allgemeinen Formel R₅COOR₆, in der R₅ und R₆ unabhängig voneinander Alkyl-, Aryl- oder Aralkylgruppen bedeuten, Carbonsäureester von mehrwertigen Alkoholen, beispielsweise Glycerintriacylate wie Glycerintriacetat, Glycerintributyrat, Carbonsäureanhydride, wie etwa in EP-A-0 269 453 geoffenbart, oder Vinylester, etwa gemäß EP-A-0 321 918 eingesetzt werden. Bevorzugt wird als Veresterungsmittel ein Carbonsäureester der allgemeinen Formel R₅COOR₆, in der R₅ und R₆ unabhängig voneinander eine Alkylgruppe mit 1 bis 6 C-Atomen bedeuten, oder ein Glycerintriacylat, ein Vinylester der allgemeinen Formel CH₂=CH-O-CO-R₇, in der R₇ Wasserstoff, eine Alkylgruppe mit 1 bis 18 C-Atomen oder eine Phenylgruppe, besonders bevorzugt eine Alkylgruppe mit 1 bis 6 C-Atomen bedeutet, ein Carbonsäureanhydrid der allgemeinen Formel R₈-CO-O-CO-R₉, in der R₈ und R₉ gleich oder ungleich, bevorzugt gleich sind und eine Alkyl-, Aryl- oder Aralkylgruppe, besonders bevorzugt eine Alkylgruppe mit 1 bis 6 C-Atomen bedeuten, eingesetzt. Ganz besonders bevorzugt werden als Veresterungsmittel Essigsäureanhydrid, Propionsäureanhydrid, Vinylacetat, Vinylbutyrat, Essigsäureäthylester, Glycerintriacetat oder Glycerintributyrat eingesetzt.
Als Verdünnungsmittel kommen in diesem Fall inerte Verdünnungsmittel, etwa aliphatische oder aromatische Kohlenwasserstoffe wie Hexan, Toluol, Xylole, Äther wie Diäthyläther, Diisopropyläther, tert.-Butyl-methyläther, Ketone wie tert.-Butyl-methylketon, ferner das Veresterungsmittel selbst oder Mischungen von oben angeführten Verdünnungsmitteln in Frage.
Pro Äquivalent der racemischen Mischung mit der allgemeinen Formel I, in der R Wasserstoff bedeutet, wird zumindest ein halbes Äquivalent, bevorzugt 1 bis 8 Äquivalente Veresterungsmittel eingesetzt. Im allgemeinen werden mit einem Überschuß des Veresterungsmittel bessere Resultate erzielt. Besonders bevorzugt wird das Veresterungsmittel gleichzeitig auch als Verdünnungsmittel eingesetzt, wobei das Veresterungsmittel in einem sehr hohen Überschuß eingesetzt wird.
Falls als Veresterungsmittel ein Carbonsäureanhydrid eingesetzt wird, wird der Reaktionsmischung zur Bindung der entstehenden Säure eine Base, etwa Kalium- oder Natriumhydrogencarbonat zugesetzt.
Die Lösung oder Suspension des racemischen Ausgangsgemisches wird sodann mit einer Hydrolase in Kontakt gebracht. Unter Hydrolasen sind z. B. Lipasen, Esterasen, Proteasen zu verstehen. Bevorzugt werden Lipasen oder Esterasen, ganz bevorzugt Lipasen oder Esterasen der Mikroorganismen Alcaligenes, Pseudomonas, Candida, Mucor eingesetzt. Die Hydrolase kann in Form gereinigter Enzymfraktionen oder in Form einer Mikroorganismussuspension, die die Hydrolase enthält, eingesetzt werden, wird aber bevorzugt in Form gereinigter Enzymfraktionen eingesetzt. Die Hydrolase kann als solche oder immobilisiert, das heißt physikalisch oder chemisch an einen Träger gebunden, eingesetzt werden.
Hydrolasen sind käuflich erwerbbar und werden bezüglich der Reaktionsbedingungen vorteilhafterweise entsprechend den Hinweisen des Anbieters eingesetzt.
Bei der erfindungsgemäßen Umsetzung erleidet die Hydrolase praktisch keinen nennenswerten Aktivitätsverlust und kann daher wiederholt eingesetzt werden.
Die geeignete Menge der Hydrolase hängt von der chemischen Beschaffenheit der verwendeten Ausgangsverbindung, der verwendeten Hydrolase, des verwendeten Verdünnungsmittels und des gegebenenfalls verwendeten Veresterungsmittel ab und kann leicht durch Vorversuche ermittelt werden. Da die eingesetzte Hydrolase wiederverwendbar ist, kann in Fällen, in denen eine große Hydrolasemenge einen günstigen Einfluß auf die Reaktionsgeschwindigkeit hat, unbedenklich eine große Hydrolasemenge eingesetzt werden, ohne dadurch die Verfahrenskosten nennenswert zu verteuern. Bevorzugt werden pro Gramm der Ausgangsverbindung der allgemeinen Formel I etwa 0,05 bis 2 g Hydrolase eingesetzt.

Da Hydrolasen sowohl Esterbindungen knüpfen, als auch wieder lösen können, können sie sowohl die selektive Hydrolyse von Verbindungen der allgemeinen Formel I, in der R eine Acylgruppe, als auch die selektive Veresterung von Verbindungen der allgemeinen Formel I, in der R Wasserstoff bedeutet, ausführen.
Die Hydrolase wird entweder der Reaktionsmischung zugegeben, oder die Reaktionsmischung wird über die Hydrolase gepumpt.
Als Reaktionstemperatur wird vorteilhafterweise jene Temperatur gewählt, bei der das Enzym seine höchste Aktivität zeigt. Diese Temperatur ist bei käuflichen Hydrolasen im allgemeinen angegeben und sonst durch einfache Vorversuche leicht zu ermitteln. Die Reaktionstemperatur beträgt je nach verwendeter Hydrolase und je nach verwendetem Substrat von -10°C bis zur Desaktivierungstemperatur der eingesetzten Hydrolase. Im allgemeinen liegen die Reaktionstemperaturen zwischen Raumtemperatur und 60°C.

Durch das Inkontaktbringen der Hydrolase mit dem racemischen Gemisch der Verbindung der allgemeinen Formel I und gegebenenfalls dem Veresterungsmittel, entsteht aus dem racemischen Ausgangsgemisch ein Reaktionsgemisch, das ein enantiomerenreines beta-Hydroxy-delta-valerolacton und ein enantiomerenreines beta-Acyloxy-delta-valerolacton enthält.
Völlig unerwarteterweise verläuft die Reaktion dabei insbesondere bei der Veresterung mit einem Vinylester in sehr kurzer Zeit und mit praktisch 100%iger Selektivität, da sich gezeigt hat, daß die Reaktion nach Umsetzung des einen Enantiomeren von selbst praktisch völlig zum Stillstand kommt. Die Umsetzung kann daher durch einfache Dünnschicht- oder Gaschromatographie verfolgt werden und es ist nicht nötig, wie bei weniger selektiven Reaktionen, die Reaktion bei einem bestimmten Umsetzungsgrad willkürlich abzubrechen, um ein Überreagieren und eine Verunreinigung des gewünschten Produktes zu verhindern. Da Enzyme nur sehr selten eine derart hohe Selektivität aufweisen und da insbesondere von Lipasen bekannt ist, daß sie bei Umsetzungen zwar ein Enantiomer bevorzugt, daß sie jedoch im allgemeinen auch das zweite Enantiomer umsetzen, sobald das Substrat an bevorzugtem Enantiomer verarmt ist, ist die hohe Selektivität der erfindungsgemäßen Reaktion in höchstem Maß erstaunlich.

Aus dem Reaktionsgemisch wird anschließend das gewünschte Enantiomer abgetrennt. Da das eine Enantiomer in der Reaktionsmischung eine Verbindung mit einer freien und das zweite Enantiomer eine Verbindung mit einer acylierten Hydroxygruppe ist, ist die Abtrennung sehr einfach durchzuführen und kann durch bekannte Methoden wie Kristallisation, Extraktion, Destillation, Chromatographie durchgeführt werden.
Als besonders überraschend hat sich bei den Verbindungen, in denen R₁ eine Hexyl- und R₂ eine Undecylgruppe bedeutet, herausgestellt, daß sich das Gemisch der enantiomerenreinen Hydroxy- und Acyloxyverbindungen durch Kristallisation auftrennen läßt, wobei die Verbindung mit der freien Hydroxygruppe vorwiegend auskristallisiert, während die Verbindung mit der acylierten Hydroxygruppe vorwiegend in Lösung bleibt. Durch das Abfiltrieren des Kristallisates wird die enantiomerenreine Acetoxyverbindung in der Mutterlauge erhalten.
Enantiomerenreine Verbindungen der allgemeinen Formel I, in der R eine Acylgruppe bedeutet und in der R₁ und R₂ die oben angegebene Bedeutung hat, mit Ausnahme von (2S, 3S, 5R)-2-Hexyl-3-benzoyloxy-5-undecyl-delta-valerolacton, das in EP-A-0 443 449 beschrieben ist, sind neu und ebenfalls Gegenstand der Erfindung.

Die aufgetrennten Verbindungen können anschließend gegebenenfalls durch übliche Methoden, wie Kristallisieren, Umkristallisieren, Chromatographie noch weiter gereinigt werden.

In einer bevorzugten Ausführungsform der Erfindung wird ein racemisches Gemisch einer Verbindung der allgemeinen Formel I, in der R₁ und R₂ Alkylgruppen mit 4 bis 20 C-Atomen und R eine Acylgruppe bedeuten, in Natriumphosphatpuffer bei pH 7 unter Zugabe eines organischen Verdünnungsmittels suspendiert. Die Reaktionsmischung wird bei Temparaturen von Raumtemperatur bis 60°C mit einer Lipase in Kontakt gebracht, wobei die Lipase entweder der Reaktionsmischung zugegeben wird, oder die Reaktionsmischung wird kontinuierlich über eine, im Reaktionsgemisch unlösliche, bevorzugt über eine immobilisierte Lipase gepumpt. Der pH-Wert der Reaktionsmischung wird durch Zugabe wäßriger Base in etwa konstant gehalten. Der Reaktionsverlauf wird chromatographisch oder anhand der Menge an verbrauchter Base verfolgt. Das erhaltene Gemisch, das ein praktisch enantiomerenreines beta-Hydroxy-delta-valerolacton und ein praktisch enantiomerenreines beta-Acyloxy-delta-valerolacton enthält, wird durch einfaches Abkühlen der Reaktionsmischung, wobei die Hydroxyverbindung kristallin ausfällt, während die Acyloxyverbindung in Lösung bleibt, aufgetrennt.

In einer anderen bevorzugten Ausführungsform der Erfindung wird ein racemisches Gemisch einer Verbindung der allgemeinen Formel I, in der R Wasserstoff und R₁ und R₂ Alkylgruppen mit 4 bis 20 C-Atomen bedeuten, in einem Carbonsäureester der allgemeinen Formel R₅COOR₆, einem Vinylalkanoat der allgemeinen Formel CH₂=CH-O-COR₇ oder einem Carbonsäureanhydrid der allgemeinen Formel R₈-CO-O-CO-R₉, in der R₅, R₆, R₇ ,R₈ und R₉ eine Alkylgruppe mit 1 bis 6 C-Atomen bedeuten, oder in einem Glycerintriacylat, besonders bevorzugt in Glycerintriacetat, Glycerintributyrat, Vinylacetat, Vinylbutyrat, Essigsäureanhydrid, Propionsäureanhydrid oder Essigsäureethylester, gegebenenfalls unter Verwendung eines inerten Verdünnungsmittels, gelöst oder suspendiert. Im Falle der Verwendung eines Carbonsäureanhydrids wird eine Base, etwa Kaliumhydrogencarbonat, zugegeben, um den pH-Wert der Reaktion konstant zu halten. Der Verlauf der Umsetzung wird chromatographisch verfolgt. Nach erfolgter Umsetzung wird die Reaktionsmischung abgekühlt, wobei das praktisch enantiomerenreine beta-Hydroxy-delta-valerolacton kristallin aus der Reaktionsmischung ausfällt oder das Verdünnungsmittel und das Veresterungsmittel werden am Rotavapor abgedampft und aus dem Rückstand das beta-Hydroxy-delta-valerolacton durch Kristallisieren oder Umkristallisieren vom beta-Acyloxy-delta-valerolacton abgetrennt.

Das Verfahren liefert reine Enantiomere von beta-Hydroxy- und beta-Acyloxy-delta-valerolactonen. Dabei hängt es von der Spezifität der verwendeten Hydrolase ab, ob das (2R, 3R, 5S)-Enantiomer oder das (2S, 3S, 5R)-Enantiomer im racemischen Gemisch umgesetzt wird. In jedem Fall entsteht ein Gemisch, das ein Enantiomer als Hydroxyverbindung und das zweite Enantiomer als Acyloxyverbindung enthält, da von der Hydrolase im Fall daß R in der allgemeinen Formel I eine Acylgruppe bedeutet, nur das (2S, 3S, 5R)-Enantiomer oder nur das (2R, 3R, 5S)-Enantiomer hydrolysiert und im Fall, daß R in der allgemeinen Formel I Wasserstoff bedeutet, nur das (2S, 3S, 5R)-Enantiomer oder nur das (2R, 3R, 5S)-Enantiomer acyliert wird, während das jeweils andere Enantiomer unreagiert in der Reaktionsmischung verbleibt. Hydroxyverbindungen können von Acyloxyverbindungen leicht nach bekannten Methoden abgetrennt werden. Nach der Trennung kann sowohl das reagierte, als auch das unreagierte Enantiomer weiterverwendet werden. Fällt ein gewünschtes Enantiomer als acyliertes Produkt an, kann es durch Abspaltung der Acylgruppe, beispielsweise durch alkalische Hydrolyse, leicht in ein gewünschtes enantiomerenreines beta-Hydroxy-valerolacton umgewandelt werden.

Ein gemäß der vorliegenden Erfindung aus dem Racemat abgetrenntes enantiomerenreines (2S, 3S, 5R)-2-Hexyl-3-hydroxy-5-undecyl-valerolacton kann zur Herstellung von N-Formyl-L-Leucin-(S)-1-(((2S, 3S)-3-hexyl-4-oxooxetan-2-yl)-methyl)-dodecylester, der unter dem Trivialnamen Tetrahydrolipstatin (THL) als Lipasehemmer bekannt ist, verwendet werden. Die Herstellung von THL aus enantiomerenreinem (2S, 3S, 5R)-2-Hexyl-3-hydroxy-5-undecyl-valerolacton ist in EP-A-0 443 449 beschrieben. Die Verwendung des erfindungsgemäßen Verfahrens in einem Verfahren zur Herstellung von THL ist ebenfalls Gegenstand der Erfindung.

Die Racemattrennung im erfindungsgemäßen Verfahren zur Herstellung von THL erfolgt in einer sehr frühen Stufe der Reaktionsfolge, wodurch das unbrauchbare Enantiomer weniger häufig umgesetzt werden muß. Das erfindungsgemäße Verfahren stellt daher und aufgrund der hohen Reaktionsgeschwindigkeit und der hohen Selektivität eine Bereicherung der Technik dar.

### Beispiel 1

0,1 g rac-(2RS, 3RS, 5SR)-2-Hexyl-3-hydroxy-5-undecyl-delta-valerolacton (0,33 mMol) (IUPAC: rac-(l,u)-3-hexyl-4-hydroxy-6-undecyl-3,4,5,6-tetrahydropyran-2-on) wurden in 2 ml Vinylacetat suspendiert und mit 0,1 g Lipase aus Candida cylindracea versetzt. Die Inkubation erfolgte bei Raumtemperatur auf einem Schüttler bei 230 UpM. Der Reaktionsverlauf wurde dünnschichtchromatographisch verfolgt.
Nach 2 Stunden war das (2R, 3R, 5S)-Enantiomer vollständig in das entsprechende (2R, 3R, 5S)-2-Hexyl-3-acetoxy-5-undecyl-delta-valerolacton umgesetzt und die Reaktion zum Stillstand gekommen.
Der optische Drehwert des Acetates, alpha_{D}²⁰ betrug -65°, der Schmelzpunkt 93°C; das entspricht den theoretischen Werten.

### Beispiel 2

wurde wie Beispiel 1 ausgeführt, wobei als Lipase 0,1 g einer Lipase aus Pseudomonas verwendet wurde. Die Ergebnisse entsprachen jenen des Beispiels 1.

### Beispiel 3

wurde wie Beispiel 1, aber unter Verwendung von 1 g Racemat, 15 ml Vinylacetat und 1 g Lipase aus Candida cylindracea durchgeführt. Nach 4 Stunden war die Reaktion zum Stillstand gekommen, die Lipase wurde abfiltriert, das Verdünnungsmittel am Rotavapor abdestilliert und der Rückstand chromatographisch getrennt (Kieselgel 60, Eluationsmittel Diisopropyläther: n-Hexan = 2:1).
Dabei wurde (2R, 3R, 5S)-2-Hexyl-3-acetoxy-5-undecyl-delta-valerolacton mit einem alpha_{D}²⁰ von -65,3° und einem Schmelzpunkt von 93°C und (2S, 3S, 5R)-2-Hexyl-3-hydroxy-5-undecyl-delta-valerolacton mit einem alpha_{D}²⁰ von +49,7° (theoretisch: +48 bis +50°) und einem Schmelzpunkt von 108°C (theoretisch: 106 - 108°C) erhalten.

### Beispiel 4

wurde wie Beispiel 3 ausgeführt, wobei als Lipase 1 g einer Lipase aus Pseudomonas eingesetzt wurde. Die Ergebnisse entsprachen jenen des Beispiels 3.

### Beispiel 5

300 ml einer 5 Gew.%igen Lösung von rac-(2RS, 3RS, 5SR)-2-Hexyl-3-hydroxy-5-undecyl-delta-valerolactons in Vinylacetat wurde bei einer Temperatur von 40°C durch ein mit 10 g Lipase aus Pseudomonas gefülltes Modul gepumpt. Der Reaktionsverlauf wurde nach Derivatisieren der Proben mit N,O-Bis-(trimethylsilyl)-trifluoracetamid gaschromatographisch verfolgt.
Nach 4,75 Stunden war das (2R, 3R, 5S)-Enantiomer praktisch vollständig in das 3-Acetoxyderivat übergeführt und die Reaktion zum Stillstand gekommen. Die Lipase wurde mit Vinylacetat gewaschen und die Reaktionsmischung auf 10°C gekühlt.
Dabei fiel das (2S, 3S, 5R)-2-Hexyl-3-hydroxy-5-undecyl-delta-valerolacton in einer Ausbeute von 4,5 g, das sind 60 % der Theorie, kristallin aus. Die Reinheit betrug mehr als 96 %.

### Beispiel 6

wurde wie Beispiel 3, aber unter Verwendung von 50 g Racemat, 1100 ml Vinylacetat und 20 g Lipase aus Pseudomonas durchgeführt.
Nach 5,25 Stunden war ein Umsatz von fast 50 % erreicht.
Die Ausbeute an kristalliner (2S, 3S, 5R)-Hydroxyverbindung betrug 12,5 g, das sind 50 % der Theorie, mit einem alpha_{D}²⁰ von +49°.

### Beispiel 7

5,0 g rac-(2RS, 3RS, 5SR)-2-Hexyl-3-hydroxy-5-undecyl-delta-valerolacton wurden in 102 ml Vinylacetat bei 40° C gelöst und nach Zugabe von 0,5 g Lipase aus Alcaligenes unter Rühren inkubiert. Der Reaktionsverlauf wurde dünnschichtchromatographisch verfolgt.
Nach 3 Stunden war das (2R, 3R, 5S)-Enantiomer vollständig in das entsprechende (2R, 3R, 5S)-2-Hexyl-3-acetoxy-5-undecyl-delta-valerolacton umgesetzt und die Reaktion zum Stillstand gekommen. Nach Filtrieren wurde die Reaktionslösung auf 10° C abgekühlt und der erhaltene Niederschlag aus Vinylacetat umkristallisiert. Dabei wurde (2S, 3S, 5R)-2-Hexyl-3-hydroxy-5-undecyl-delta-valerolacton in einer Ausbeute von 60% der Theorie und in 97%iger Reinheit erhalten.

### Beispiel 8

wurde wie Beispiel 3, aber unter Verwendung von 300 ml Essigsäureäthylester anstatt Vinylacetat, unter Zugabe von 2 Äquivalenten Vinylacetat bezogen auf das Racemat und unter Verwendung von 5 g Lipase aus Pseudomonas durchgeführt.
Nach 30 Stunden war das (2R, 3R, 5S)-Enantiomer praktisch vollständig in das (2R, 3R, 5S)-Acetoxyderivat übergeführt. Nach Abkühlen der Reaktionsmischung auf 10°C fiel das kristalline (2S, 3S, 5R)-2-Hexyl-3-hydroxy-5-undecyl-delta-valerolacton in einer Ausbeute von 25 % der Theorie mit einem alpha_{D}²⁰ von +47,1° und einem Schmelzpunkt von 106°C an.

### Beispiel 9

300 ml einer 5 Gew.%igen Lösung von rac-(2RS, 3RS, 5SR)-2-Hexyl-3-hydroxy-5-undecyl-delta-valerolacton in Ethylacetat wurden mit 6 Äquivalenten Vinylacetat bezogen auf das Racemat und 5 g Lipase aus Pseudomonas versetzt und bei 40°C unter Rühren inkubiert. Nach 15 Stunden war das (2R, 3R, 5S)-Enantiomer praktisch vollständig in das (2R, 3R, 5S)-3-Acetoxyderivat übergeführt. Die Lipase wurde abfiltriert und die Reaktionsmischung auf 10°C abgekühlt.
Dabei fiel das (2S, 3S,5R)-2-Hexyl-3-hydroxy-5-undecyl-delta-valerolacton in einer Ausbeute von 25 % mit einem alpha_{D}²⁰ von +48,4° und einem Schmelzpunkt von 106°C kristallin an.

### Beispiel 10

wurde wie Beispiel 8 aber unter Verwendung einer Lösung des Racemates in tert.-Butyl-methyläther anstatt in Essigsäureäthylester, von 6 Äquivalenten Vinylacetat anstatt 2 Äquivalenten und von 10 g Lipase aus Pseudomonas, anstatt 5 g ausgeführt.
Nach 16,75 Stunden war das (2R, 3R, 5S)-Enantiomer praktisch vollständig in das (2R, 3R, 5S)-Acetoxyderivat übergeführt. Die Ausbeute betrug 2,1 g, das sind 28 % der Theorie, alpha_{D}²⁰ betrug +48°.

### Beispiel 11

625,0 g rac-(2RS, 3RS, 5SR)-2-Hexyl-3-hydroxy-5-undecyl-delta-valerolacton wurden bei 40°C in 4688 ml Toluol gelöst, mit 1120 ml Vinylbutyrat versetzt und die Lösung durch ein mit 150 g Lipase aus Alcaligenes gefülltes Modul gepumpt. Der Reaktionsverlauf wurde wie im Beispiel 5 beschrieben, verfolgt.
Nach 5,8 Stunden war das (2R, 3R, 5S)-Enantiomer praktisch vollständig in das 3-Butyroylderivat übergeführt. Das Modul wurde mit 1000 ml Toluol gespült, die Waschlösung auf ein Minimum eingeengt, zur Reaktionslösung gegeben und das Gemisch auf 4°C abgekühlt.
Dabei wurden 261,1 g, das sind 83,6 % der Theorie, kristallines (2S, 3S, 5R)-2-Hexyl-3-hydroxy-5-undecyl-delta-valerolacton mit einer chemischen Reinheit von mehr als 97 % und einer Enantiomerenreinheit von mehr als 99,5 % erhalten.

### Beispiel 12:

Wurde wie Beispiel 11, aber unter Verwendung von 123 ml Vinylbutyrat anstatt 1120 ml und 10,0 g eines Immobilisates einer Cholesterin-Esterase aus Pseudomonas auf einem anorganischen Träger, anstatt 150 g der Alcaligenes Lipase, durchgeführt, wobei die Reaktion nach 25,9 Stunden beendet war.
Dabei wurden 251,7 g das sind 80,5 % der Theorie kristallines (2S, 3S, 5R)-2-Hexyl-3-hydroxy-5-undecyl-delta-valerolacton mit einer chemischen Reinheit von mehr als 98 % und einer Enantiomerenreinheit von mehr als 97,5 % erhalten.

### Beispiel 13:

Wurde wie Beispiel 11, aber unter Verwendung von 20,9 g racemischem Ausgangsgemisch anstatt 625,0 g, 156,3 ml Toluol anstatt 4688 ml, 37,4 ml Vinylbutyrat anstatt 1120 ml und 5 g einer Pseudomonas Lipase, die auf einem anorganischen Träger immobilisiert war, anstatt 150 g der Alcaligenes Lipase, durchgeführt, wobei die Reaktion nach 5 Stunden beendet war. Das Modul wurde mit 100 ml Toluol anstatt mit 1000 ml gespült.
Dabei wurden 10,0 g das sind 95,4 % der Theorie, kristallines (2S, 3S, 5R)-2-Hexyl-3-hydroxy-5-undecyl-delta-valerolacton mit einer chemischen Reinheit von mehr als 97 % und einer Enantiomerenreinheit von mehr als 99,5 % erhalten.

### Beispiel 14

300 ml einer 5 Gew.%igen Lösung von rac-(2RS, 3RS, 5SR)-2-Hexyl-3-hydroxy-5-undecyl-delta-valerolacton in Toluol wurden nach Zugabe von je einem Äquivalent Essigsäureanhydrid und getrocknetem, pulverisiertem KHCO₃ bei 40° C über ein mit 5 g Lipase aus Pseudomonas gefülltes Modul gepumpt. Der Reaktionsverlauf wurde dünnschichtchromatographisch verfolgt. Nach erfolgter Umsetzung wurde das Reaktionsgemisch filtriert und mit verdünnter, wäßriger NaHCO₃-Lösung extrahiert. Die organische Phase wurde über Natriumsulfat getrocknet und anschließend auf 10° C abgekühlt.
Dabei fielen 3,5 g (2S, 3S, 5R)-2-Hexyl-3-hydroxy-5-undecyl-delta-valerolacton, das ist eine Ausbeute von 46% der Theorie, mit einer Reinheit von 93 % an.

### Beispiel 15

400 ml einer 2,5 Gew.%igen Lösung von rac-(2RS, 3RS, 5SR)-2-Hexyl-3-hydroxy-5-undecyl-delta-valerolacton in Essigsäureäthylester wurde bei einer Temperatur von 40°C über ein mit 5 g Lipase aus Pseudomonas gefülltes Modul gepumpt. Das bei der Reaktion entstehende Äthanol wurde kontinuierlich abdestilliert.
Der Reaktionsverlauf wurde dünnschichtchromatographisch verfolgt.
Nach erfolgter Umsetzung wurde das Verdünnungsmittel abgedampft. Der Rückstand wurde aus Vinylacetat umkristallisiert. Dabei wurden 2,5 g (2S, 3S, 5R)-2-Hexyl-3-hydroxy-5-undecyl-delta-valerolacton, das sind 50 % der Theorie, mit einer Reinheit von 97 % erhalten.

### Beispiel 16

wurde wie Beispiel 15, aber unter Verwendung von 25 g Racemat, 350 ml Essigsäureäthylester und 10 g Lipase aus Pseudomonas bei 50 °C durchgeführt.
Nach erfolgter Umsetzung wurde die Reaktionslösung abgekühlt, wobei 10,4 g einer kristallinen Mischung aus 73 % (2S, 3S, 5R)-2-Hexyl-3-hydroxy-5-undecyl-delta-valerolacton und 27% (2R, 3R, 5S)-2-Hexyl-3-acetoxy-5-undecyl-delta-valerolacton erhalten wurden. Nach Umkristallisieren aus Essigsäureäthylester wurde die (2S, 3S, 5R)-Hydroxyverbindung mit einer Reinheit von 97 % erhalten.

### Beispiel 17

1 g rac-(2RS, 3RS, 5SR)-2-Hexyl-3-acetoxy-5-undecyl-delta-valerolacton, erhalten durch Veresterung von rac-(2RS, 3RS, 5SR)-2-Hexyl-3-hydroxy-5-undecyl-delta-valerolacton mit Essigsäureanhydrid in Gegenwart von Dimethylaminopyridin in Pyridin, wurde in 60 ml 0,01 molarem Natriumphosphatpuffer, pH = 7,0 und 3 ml Tetrahydrofuran suspendiert. Nach Zugabe von 0,5 g Lipase aus Pseudomonas wurde das Reaktionsgemisch bei 40 °C inkubiert, wobei der pH-Wert durch kontinuierliche Zugabe einer wäßrigen, 0,1 m Natriumhydroxidlösung konstant gehalten wurde. Die Reaktion wurde dünnschichtchromatographisch und anhand der verbrauchten Natriumhydroxidlösung verfolgt.
Nach etwa 40 Stunden war ein Umsatz von 50 % bezogen auf das racemische Ausgangsprodukt erreicht und die Reaktion zum Stillstand gekommen.
Die Reaktionsmischung wurde mit Essigsäureäthylester ausgeschüttelt und die organische Phase über Natriumsulfat getrocknet und abgedampft. Der Rückstand wurde aus Vinylacetat umkristallisiert.
Dabei wurden 0,3 g (2R, 3R, 5S)-2-Hexyl-3-hydroxy-5-undecyl-delta-valerolacton, das sind 60 % der Theorie, mit einem alpha_{D}²⁰ von -48,2° erhalten. Der optische Drehwert alpha_{D}²⁰ von (2S, 3S, 5R)-2-Hexyl-3-acetoxy-delta-valerolacton betrug +65°.

### Beispiel 18

2,0 g (5,65 mMol) rac-(2RS, 3RS, 5SR)-2-Hexyl-3-hydroxy-5-undecyl-delta-valerolacton wurden in 10 ml Toluol bei 40 °c gelöst und mit 5 g Propionsäureanhydrid, 1 g Natriumhhydrogencarbonat und 0,5 g Lipase aus Pseudomonas versetzt. Die Inkubation erfolgte bei 40 °C auf einem Schüttler mit 230 UpM.
Nach 3 Stunden war ein Umsatz von nahezu 50 % erreicht. Nach Abfiltrieren wurde die Reaktionsmischung auf 10 °C abgekühlt, wobei 480 mg, das sind 48% der Theorie, kristallines, reines (2S, 3S, 5R)-2-Hexyl-3-hydroxy-5-undecyl-delta-valerolacton erhalten wurden .

### Beispiel 19

wurde wie Beispiel 18, aber unter Verwendung von 0,5 g Lipase aus Alcaligenes als Hydrolase durchgeführt.
Nach 2 Stunden war ein Umsatz von nahezu 50% erreicht. Nach Abfiltrieren wurde die Reaktionsmischung auf 10 °C abgekühlt, wobei 0,5 g, das sind 50 % der Theorie kristallines (2S, 3S, 5R)-2-Hexyl-3-hydroxy-5-undecyl-delta-valerolacton, das mit 1,5 % des entsprechenden (2R, 3R, 5S)-Propionates verunreinigt war, erhalten wurde.

### Beispiel 20

2,0 g rac-(2RS, 3RS, 5SR)-2-Hexyl-3-hydroxy-5-undecyl-delta-valerolacton (5,65 mMol) wurden in 10 ml Toluol bei 40 °C gelöst und mit 5 g Glycerintributyrat und 0,5 g Lipase aus Pseudomonas versetzt. Die Inkubation erfolgte bei 40 °C auf einem Schüttler mit 230 UpM.
Nach 48 Stunden war ein Umsatz von 50 % erreicht. Nach Abfiltrieren des Enzyms und Verdünnen der Reaktionsmischung mit 10 ml Toluol wurden 750 mg, das sind 75 % der Theorie, (2R, 3R, 5S)-2-Hexyl-3-hydroxy-5-undecyl-delta-valerolacton, verunreinigt mit 5 % des (2R, 3R, 5S)-Butyrates durch Abkühlen der Reaktionsmischung auf 0 °C kristallin erhalten.

### Beispiel 21

wurde wie Beispiel 20, aber unter Verwendung von 0,5 g Lipase aus Alcaligenes durchgeführt.
Nach 48 Stunden war ein Umsatz von 50 % erreicht. Das Enzym wurde abfiltriert und die Reaktionsmischung auf 10°C abgekühlt, wobei 50 % der Theorie kristallines (2S, 3S, 5R)-2-Hexyl-3-hydroxy-5-undecyl-delta-valerolacton, das mit 1,5 % des entsprechenden (2R, 3R, 5S)-Butyrates verunreinigt war, erhalten wurden.

### Beispiel 22

wurde wie Beispiel 20, aber unter Verwendung von 5 g Glycerintriacetat als Veresterungsmittel durchgeführt.
Nach 24 Stunden war ein Umsatz von 50% erreicht. Das Enzym wurde abfiltriert und die Reaktionsmischung auf 10°C abgekühlt, wobei 890 mg eines Kristallisates, das 73% enantiomerenreines (2S, 3S, 5R)-2-Hexyl-3-hydroxy-5-undecyl-delta-valerolacton und 27 % des entsprechenden (2R, 3R, 5S)-Acetates enthielt, erhalten wurden. Nach Umkristallisieren aus tert.-Butyl-methyläther wurden 78 % der im Kristallisat enthaltenen (2S, 3S, 5R)-Hydroxyverbindung mit einem alpha_{D}²⁰ von +48° erhalten.

### Beispiel 23

wurde wie Beispiel 22, aber unter Verwendung von 0,5 g Lipase aus Alcaligenes durchgeführt.
Nach 24 Stunden war ein Umsatz von 50 % erreicht. Das Enzym wurde abfiltriert und die Reaktionsmischung auf 10°C abgekühlt, wobei 630 mg, das sind 63 % der Theorie, kristallines (2S, 3S, 5R)-2-Hexyl-3-hydroxy-5-undecyl-delta-valerolacton mit einer Reinheit von 93 % erhalten wurden.

### Beispiel 24

3 g einer kristallinen Mischung von 77 % (2S, 3S, 5R)-2-Hexyl-3-hydroxy-5-undecyl-delta-valerolacton mit 23% von (2R, 3R, 5S)-2-Hexyl-3-acetoxy-5-undecyl-delta-valerolacton wurden aus tert.-Butyl-methyläther umkristallisiert. Dabei wurden 1,8 g (2S, 3S, 5R)-2-Hexyl-3-hydroxy-5-undecyl-delta-valerolacton, das sind 78% bezogen auf das eingesetzte Enantiomer, mit einem alpha_{D}²⁰ von +48° erhalten.

### Beispiel 25

15,89 g eines äquimolaren kristallinen Gemisches von (2S, 3S, 5R)-2-Hexyl-3-hydroxy-5-undecyl-delta-valerolacton und (2R, 3R, 5S)-2-Hexyl-3-acetoxy-5-undecyl-delta-valerolacton wurden aus 300 ml Toluol umkristallisiert. Dabei wurden 3,5 g, das sind 46 % der Theorie, (2S, 3S, 5R)-2-Hexyl-3-hydroxy-5-undecyl-delta-valerolacton in 97%iger Reinheit erhalten.

Die Dünnschichtchromatographie, die in den Beispielen beschrieben ist, wurde in Diisopropyläther:n-Hexan = 2:1, Cersulfat-Sprühreagens, ausgeführt. Der optische Drehwert alpha_{D}²⁰ wurde in Chloroformlösung (c = 1 g/100 ml) bestimmt. Die Ausbeuten, die in den Beispielen angegeben sind, sind stets auf die eingesetzte Menge des reinen Enantiomers im racemischen Ausgangsgemisch bezogen.
Die Enantiomerenreinheit von (2S, 3S, 5R)- und von (2R, 3R, 5S)-2-Hexyl-3-acetoxy-5-undecyl-delta-valerolacton wurde mittels ¹HNMR unter Verwendung von Tris(3-(2,2,2-trifluoro-1-hydroxyethyliden)-d-camphorato)-europium bestimmt. Der optische Drehwert alpha_{D}²⁰ beträgt für die (2R, 3R, 5S)-Verbindung - 65° und für die (2S, 3S, 5R)-Verbindung +65°. Der Schmelzpunkt beträgt 93 °C.

## Patentansprüche

1. Verfahren zur Trennung von racemischen Gemischen einer Verbindung der allgemeinen Formel 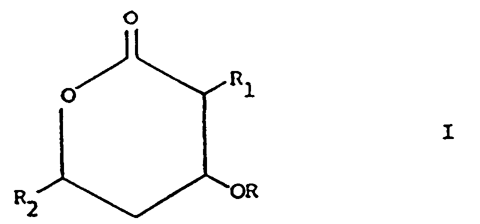 in der R Wasserstoff oder eine Acylgruppe und R₁ und R₂ unabhängig voneinander Wasserstoff, eine geradkettige oder verzweigte Alkylgruppe mit 4 bis 20 C-Atomen, die in einer anderen als der alpha- oder beta-Stellung durch ein Sauerstoffatom unterbrochen sein kann oder eine unsubstituierte oder durch unter den Reaktionsbedingungen inerte Gruppen substituierte Aralkylgruppe bedeuten, mit der Maßgabe, daß R₁ und R₂ nicht gleichzeitig Wasserstoff bedeuten, dadurch gekennzeichnet, daß das racemische Gemisch einer Verbindung der allgemeinen Formel I in einem Verdünnungsmittel vorgelegt und in Gegenwart einer Hydrolase und im Fall, daß R Wasserstoff bedeutet, in Gegenwart eines Veresterungsmittels reagieren gelassen wird, wobei ein Reaktionsgemisch entsteht, das ein enantiomerenreines beta-Hydroxy-delta-valerolacton und ein enantiomerenreines beta-Acyloxy-delta-valerolacton enthält, das auf übliche Art und Weise aufgetrennt wird.

2. Verfahren nach Anspruch 1, dadurch gekennzeichnet, daß eine Verbindung der Formel I, in der R₁ und R₂ unabhängig voneinander eine Alkylkette mit 4 bis 20 C-Atomen bedeuten, eingesetzt wird.

3. Verfahren nach einem der Ansprüche 1 oder 2, dadurch gekennzeichnet, daß eine Verbindung der Formel I eingesetzt wird, in der R Wasserstoff bedeutet.

4. Verfahren nach einem der Ansprüche 1 bis 3, dadurch gekennzeichnet, daß als Veresterungsmittel ein Carbonsäureester der allgemeinen Formel R₅COOR₆, ein Vinylalkanoat der allgemeinen Formel CH₂=CH-O-COR₇ oder ein Carbonsäureanhydrid der allgemeinen Formel R₈-CO-O-CO-R₉, in der R₅, R₆, R₇, R₈ oder R₉ eine Alkylgruppe mit 1 bis 6 C-Atomen bedeuten oder ein Glycerintriacylat eingesetzt wird.

5. Verfahren nach einem der Ansprüche 1 oder 2, dadurch gekennzeichnet, daß eine Verbindung der allgemeinen Formel I eingesetzt wird, in der R eine Acylgruppe bedeutet.

6. Verfahren nach einem der Ansprüche 1 bis 5, dadurch gekennzeichnet, daß als Hydrolase eine Lipase eingesetzt wird.

7. Verwendung des Verfahrens nach einem der Ansprüche 1 bis 6 in einem Verfahren zur Herstellung enantiomerenreiner Oxetanone.

8. Verwendung nach Anspruch 7 zur Herstellung von N-Formyl-L-leucin-(S -1-(((2S,3S)-3-hexyl-4-oxooxetan-2-yl)methyl) dodecylester.

9. Enantiomerenreine Verbindungen der allgemeinen Formel 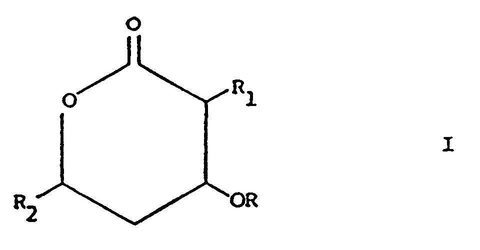 in der R eine Acylgruppe und R₁ und R₂ unabhängig voneinander Wasserstoff, eine geradkettige oder verzweigte Alkylgruppe mit 4 bis 20 C-Atomen, die in einer anderen als der alpha- oder beta-Stellung durch ein Sauerstoffatom unterbrochen sein kann oder eine unsubstituierte oder durch unter den Reaktionsbedingungen inerte Gruppen substituierte Aralkylgruppe bedeuten, mit Ausnahme von (2S, 3S, 5R)-2-Hexyl-3-benzoyloxy-5-undecyl-delta-valerolacton.

10. (2S, 3S, 5R)-2-Hexyl-3-acyloxy-5-undecyl-delta-valerolacton.
